# EUROPÄISCHE PATENTANMELDUNG

(11) **EP 2 462 859 A1**
(43) Veröffentlichungstag der Anmeldung: **13.06.2012**
(21) Anmeldenummer: 11401643.9
(22) Anmeldetag: 29.11.2011
(51) Int. Cl.: A61B 1/00, A61B 1/313

(54) **Schutzüberzug für ein Laparoskop**

(30) Priorität: 09.12.2010 DE 102010061133
(71) Anmelder: IFM-Gerbershagen GmbH, 87719 Mindelheim (DE); G & H Medical GmbH & Co. KG, 85570 Markt Schwaben (DE)
(72) Erfinder: Gerbershagen, Jochen, 87719 Mindelheim (DE); Brendel, Gerhard, 81475 München (DE); Gampenrieder, Hans, 85570 Markt Schwaben (DE)
(74) Vertreter: Eder, Christian

(57) **Zusammenfassung**

Die Erfindung betrifft einen Schutzüberzug für ein Laparoskop 11, wobei der Schutzüberzug 1 in distaler Richtung gegen die Umgebung abgeschlossen und dichtend ausgebildet ist, um im aufgeschobenen Zustand im operativen Einsatz das Innere des Abdomens vor Verunreinigungen und/oder Infektionen und/oder das Laparoskop (11) vor Verunreinigungen zu schützen, wobei der Schutzüberzug 1 einen rohrförmigen Bereich 3 aufweist, welcher auf einen Schaft 13 des Laparoskops 11 geschoben werden kann, wobei der Schutzüberzug 1 wenigstens im Bereich des Schaftes 13 als geschlossene Hülle ausgebildet ist und wobei der Schutzüberzug 1 für die Optik des Laparoskops am distalen Kopfende 19 ein Sichtfenster 9 aufweist. Weiterhin betrifft die Erfindung ein Laparoskop 11 für einen derartigen Schutzüberzug 1, sowie eine Anordnung aus einem Laparoskop mit einem derartigen Schutzüberzug 1.

## Beschreibung

Die vorliegende Erfindung betrifft einen Schutzüberzug für ein Laparoskop.

In der laparoskopischen Chirurgie werden u.a. starre Endoskope, sogenannte Laparoskope verwendet, um Eingriffe (Operationen, Diagnosen) innerhalb der Bauchhöhle vorzunehmen. Bei der Laparoskopie wird für diagnostische Untersuchungen oder für operative Eingriffe beispielsweise ein Laparoskop durch eine mit Hilfe eines Trokars geschaffene Öffnung in den Bauchraum (Abdomen) eingeführt.

Um das Eindringen von Verunreinigungen, insbesondere Bakterien und Keime, in den Bauchraum und hierdurch bedingte Infektionen des Patienten zu vermeiden, muss ein Laparoskop hochsteril sein, was üblicherweise durch Autoklavieren erfolgt.

Insbesondere bei Laparoskopen, bei welchen die Optik in Form eines CCD-Sensors distal im Endoskopkopf sitzt, kann jedoch durch die Beanspruchung (thermische und Druck-) beim Autoklavieren eine Undichtigkeit entstehen, infolge welcher Dampf in das Laparoskopinnere eindringt und wesentliche Teile des Laparoskops, insbesondere die Optik, schädigt oder gar zerstört.

Bei Laparoskopen, welche unterhalb des distalen Kopfendes einen abwinkelbaren Bereich aufweisen, könnte zudem die Mechanik zur Steuerung des Abwinkelvorgangs beeinträchtigt oder gar zerstört werden. In Anbetracht der hohen Herstellungskosten eines Laparoskops verursachen derartige Schäden nachteiligerweise hohe Kosten für Reparatur oder Ersatz.

Der vorliegenden Erfindung liegt daher die Aufgabe zugrunde, für laparoskopische Eingriffe in der Humanmedizin hochsterile Bedingungen zu ermöglichen und dennoch Schäden am Laparoskop zu vermindern oder gar zu vermeiden.

Diese Aufgabe wird erfindungsgemäß durch einen Schutzüberzug mit den Merkmalen des Patentanspruchs 1, welcher auf ein Laparoskop aufgeschoben werden kann, gelöst.

Durch das Vorsehen eines erfindungsgemäßen sterilen Schutzüberzugs, welcher vorzugsweise nur einmalig verwendet wird (Einwegüberzug), ist es vorteilhafterweise möglich, einen Eingriff unter den geforderten Bedingungen an die Sterilität vorzunehmen, ohne dass das Laparoskop selbst und damit dessen sensible Elektronik einem Autoklavieren ausgesetzt werden muss. Durch das Überstreifen bzw. Aufschieben des sterilen Schutzüberzuges kommt das Laparoskop bzw. dessen eindringender Schaft nicht mit dem Körperinneren direkt in Berührung, so dass Verunreinigungen oder eine Kontaminierung, insbesondere eine bakterielle Verseuchung, des Patienten ausgeschlossen werden können.

Da das Laparoskop nicht direkt mit dem Körperinneren in Berührung kommt, sind zudem auch Verunreinigungen des Laparoskops selbst, insbesondere durch Körperflüssigkeiten, ausgeschlossen.

Da ein derartiger Überzug, insbesondere als Einweg- oder gar Wegwerfartikel, vergleichsweise geringe Herstellungskosten besitzt, können hierdurch auf einfache Art und Weise Kosten in erheblicher Höhe eingespart werden. Zudem ist die Sterilität eines Einwegartikels durch entsprechende Verpackung leichter zu bewerkstelligen.

Selbstverständlich ist es aber auch denkbar, einen Schutzüberzug wiederzuverwenden; hierbei müsste allerdings der Schutzüberzug selbst sterilisierbar, insbesondere autoklavierbar sein, ohne seine Dichtigkeit zu verlieren. Das Sterilisieren könnte hierbei in einem Autoklav vor Ort geschehen, wobei auch ein Sterilisieren einmal verwendeter Schutzüberzüge in Form eines Aufbereitens in einer entsprechenden aushäusigen Servicestelle vorstellbar ist.

Um den Schaft des Laparoskops, welcher bei dem Eingriff in das Körperinnere eingeführt wird, möglichst definiert zu umgeben, weist der Schutzüberzug einen distal dichtend abgeschlossenen rohrförmigen Bereich auf, welcher auf den Schaft des Laparoskops geschoben werden kann. Dieser rohrförmige Bereich ist hierbei in seiner äußeren Kontur derart ausgebildet, dass er beim Einführen in einen Trokar bzw. dessen Tubus eine Dichtwirkung zwischen Außenumfang des rohrförmigen Bereichs und den Innenumfang des Tubus erzeugt, wie es aus dem Einführen eines herkömmlichen Laparoskops (ohne Schutzüberzug) in einen Tubus bekannt und notwendig ist. Hierbei umgibt der Schutzüberzug wenigstens im Bereich des bei dem laparoskopischen Eingriff eindringenden Bereichs des Schafts diesen als geschlossene Hülle, wobei am distalen Ende für die Optik (CCD-Sensor, Linse) ein Sichtfenster vorgesehen ist.

In bevorzugter Ausgestaltung der Erfindung ist der Schutzüberzug im aufgeschobenen bzw. installierten Zustand im Bereich eines abwinkelbaren distalen Segments des Laparoskops flexibel ausgebildet, um ein Abwinkeln des Schaftes in diesem Bereich zu ermöglichen. Hierbei ist der Schutzüberzug im aufgeschobenen Zustand im Bereich des distalen Laparoskopkopfs vorzugsweise eng anliegend ausgebildet, um eine definierte relative Lage dieses Bereichs des Schutzüberzugs zum Laparoskopkopf zu gewährleisten. Hierdurch kann vorteilhafterweise ein Spiel und/oder eine Relativbewegung zwischen Sichtfenster und Optik vermieden werden. Das Sichtfenster am distalen Ende des rohrförmigen Bereichs kann hierbei als eingefügte, beispielsweise eingeklebte oder eingepresste, Linse ausgeführt sein oder als durchsichtiger Bereich in dem Material des Schutzüberzugs, vorzugsweise einstückig, integriert sein.

In weiterer Ausgestaltung der Erfindung weist der Schutzüberzug eine Befestigungseinrichtung auf, mit welcher wenigstens der rohrförmige Bereich unter Zug am Schaft befestigbar ist. Hierdurch wird vorteilhafterweise eine ortsfeste Anordnung des Sichtfensters relativ zur Optik und ein definiertes Anliegen des Sichtfenster an der Optik ohne Spiel ermöglicht. Die Befestigungseinrichtung kann hierbei beispielsweise als Magnetverschluss, Federring oder Clip ausgebildet sein, welcher mit einem entsprechend hierzu vorzugsweise komplementär ausgebildeten Bereich des Laparoskops zusammenwirkt, um eine definierte lösbare Befestigung zu ermöglichen.

In bevorzugter Ausgestaltung der Erfindung ist der Schutzüberzug im Bereich des Griffs (dünn bzw.) flexibel und oder mit Spiel zu diesem Bereich ausgebildet, um eine Bedienung der Bedienelemente des Laparoskops, wie beispielsweise Handrad, Knöpfe, etc. durch den Schutzüberzug hindurch zu ermöglichen, so dass auch in diesem Bereich das Laparoskop durch den Schutzüberzug nach außen steril ist.

Der Schutzüberzug kann aus beliebigen, je nach Abschnitt geeigneten Materialien als Mehrkomponentenverbundstoff gefertigt sein. Beispielsweise ist es denkbar, den rohrförmigen Bereich aus Metall (Aluminium-, Stahlrohr), Glas, Kunststoff, etc. zu fertigen, wobei der distale abwinkelbare aus einem flexiblen Material (Kunststoff, Gummi, etc.) bestehende Bereich unterhalb des Laparoskopkopfes hiermit dichtend, insbesondere mittels Klebverbindung, verbunden ist. Der Bereich des Schutzüberzuges zur Abdeckung des Bediengriffes des Laparoskops, kann aus zumindest teilweise transparenter Kunststofffolie gefertigt sein, um eine Bedienung der Bedienelemente von außen auf einfache Art und Weise zu ermöglichen.

Selbstverständlich ist es aber auch denkbar, mehrere Bereiche oder den gesamten Schutzüberzug einstückig aus einem Material (Gummi, Kunststoff, etc.) mit eventuell unterschiedlicher Dicke herzustellen.

Ein erfindungsgemäßes Laparoskop mit einem an seinem starren Schaft am distalen Ende befindlichen abwinkelbaren Segment weist für einen aufschiebbaren und abnehmbaren Schutzüberzug gemäß den vorstehend erläuterten Ausgestaltungen eine Befestigungseinrichtung auf, welche mit einer an oder in dem Schutzüberzug angeordneten, hierzu komplementär ausgebildeten Befestigungseinrichtung zusammenwirkt, um den rohrförmigen Bereich am Schaft eindeutig und unter Spannung bzw. Zug zu befestigen. Als lösbare Befestigungseinrichtung kann hier beispielsweise eine, vorzugsweise umlaufende, Nut am Schaft oder Bedienteil dienen, welche mit einer Ringfeder in oder an dem Schutzüberzug und/oder einer an dessen Innenumfang an entsprechender Stelle ausgebildeten, vorzugsweise umlaufenden, Wulst zusammenwirkt.

Ebenso ist es denkbar, eine magnetische Befestigung, beispielsweise aus einem Magneten, insbesondere Ring oder Stabmagnet, und einem weiterem, insbesondere ring-oder stabförmigen, Magneten oder ferromagnetischem Material zu realisieren. Selbstverständlich ist es auch denkbar, die Befestigungseinrichtung in dem Schutzüberzug zu integrieren, beispielsweise als flexible Engstelle, wie Vorsprung oder Vertiefung, welche auch umlaufend ausgebildet sein kann. Zudem könnte auch ein Drehverschluss (Schraub- oder Bajonettverbindung, etc.) hierfür Anwendung finden, welcher jeweils zumindest als ein Teil der Befestigungseinrichtung am erfindungsgemäßen Laparoskop ausgebildet ist, um mit einem hierzu komplementär ausgebildeten Bereich des Schutzüberzugs oder einem hier angeordneten Teil einer Befestigungseinrichtung zusammenzuwirken.

Weiterhin ist von der Erfindung eine Anordnung aus einem (beliebigen) Laparoskop und einem erfindungsgemäßen Schutzüberzug in wenigstens einer der vorstehend erläuterten Ausgestaltungen umfasst.

Weitere vorteilhafte Ausgestaltungen der Erfindung ergeben sich aus den Unteransprüchen.

Die Erfindung wird nachfolgend anhand eines in der Zeichnung dargestellten Ausführungsbeispiels näher erläutert.

In der Zeichnung zeigen:
- Fig. 1: eine perspektivische Ansicht eines Schutzüberzugs nach der Erfindung;
- Fig. 2: eine perspektivische Ansicht eines Laparoskops;
- Fig. 3: eine Seitenansicht auf ein Laparoskop nach Fig. 2 mit geradem Kopf;
- Fig. 4: eine Seitenansicht auf ein Laparoskop nach Fig. 2 mit abgewinkeltem Kopf;
- Fig. 5: eine Längsschnittansicht eines Schutzüberzugs nach Fig. 1 in gebrochener Darstellung;
- Fig. 6: eine Längsschnittansicht eines auf ein Laparoskop aufgeschobenen Schutzüberzugs in gebrochener Darstellung;
- Fig. 7: eine perspektivische Ansicht eines Laparoskop nach fig. 2 gebrochener Darstellung und
- Fig. 8: eine schematische Längsschnittansicht eines auf ein Laparoskop aufgeschobenen Schutzüberzugs in abgebrochener Darstellung.

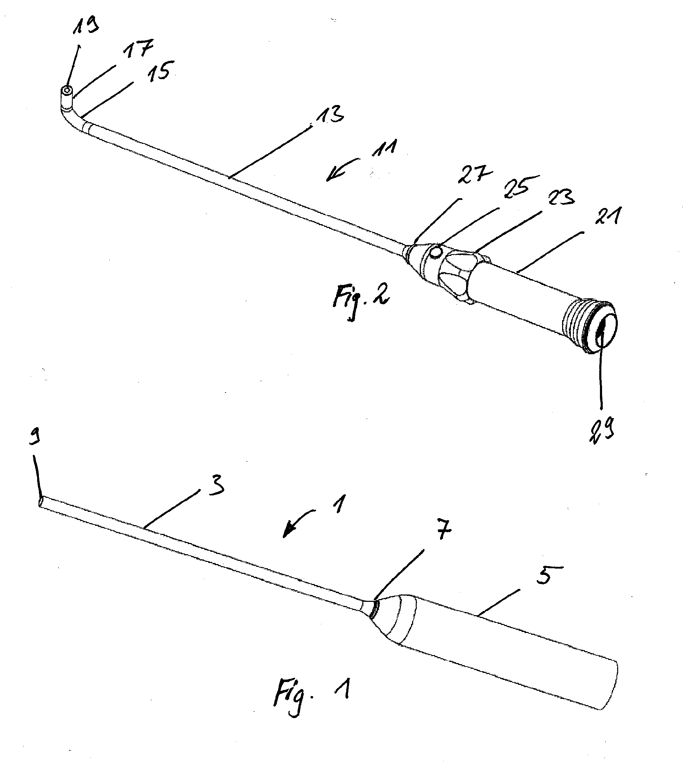

Der in Fig. 1 dargestellte Schutzüberzug 1 weist, um auf ein in Fig. 2 dargestelltes Laparoskop 11 aufgeschoben bzw. aufgezogen zu werden, einen rohrförmigen Bereich 3 aus einem formsteifen Material wie beispielsweise Kunststoff oder Metall auf, an dessen distalem Ende sich ein Sichtfenster 9 befindet. An diesen rohrförmigen Bereich mit vorzugsweise gleichbleibendem Querschnitt und Außendurchmesser schließt sich am gegenüberliegenden Ende ein im Querschnitt erweiterter proximaler Bereich 5 an, welcher zur Aufnahme eines Handgriffs bzw. Bediengriffs 21 des Laparoskops 11 dient. Der Schutzüberzug 1 weist nur am proximalen Ende und damit am proximalen Bereich 5 eine stirnseitige Öffnung auf, durch welche das Laparoskop 11 mit seinem Schaft 13 bzw. seinem Kopf 17 voraus eingeschoben bzw. eingeführt werden kann. Dagegen ist der gesamte übrige Bereich des Schutzüberzuges inklusive des Sichtfensters 9 abgeschlossen und dicht gegenüber der Umgebung ausgebildet, so dass ein hierin eingeschobenes Laparoskop 11 vor der Umgebung und damit vor Verunreinigungen geschützt ist.

Der rohrförmige Bereich entspricht hierbei der Länge des Schaftes 13, so dass der Schaft 13 in seiner gesamten Länge in den rohrförmigen Bereich eingeschoben werden kann und das Kopfende mit Deckglas, Optik, CCD-Sensor und LED-Lichtquelle stirnseitig durch das Sichtfenster 9 abgeschlossen wird. Um eine definierte Lage des Kopfendes 19 am Sichtfenster 9 zu ermöglichen, weist der Schutzüberzug 1 vorzugsweise am Übergang des rohrförmigen Bereichs 3 zu dem proximalen erweiterten Bereich 5 einen Magnetring oder einen Ring aus einem ferromagnetischen Metall 7 auf, welcher beispielsweise außen am Schutzüberzug aufgeklebt, oder von der Innenseite des Schutzüberzuges aufgeklebt, oder in das Material des Schutzüberzuges selbst integriert ist. Dieser Ring 7 wirkt mit einem am Laparoskop 11 an entsprechender Stelle angeordneten Magnetring 27 zusammen, um eine insbesondere in der Länge definierte Befestigung des rohrförmigen Bereiches 3 unter Zug am Laparoskop bzw. dessen Schaft 13 zu ermöglichen, so dass das Kopfende 19, insbesondere dessen CCD-Sensor und LED-Lichtquelle, definiert und relativ zum Sichtfenster 9 ortsfest anliegt.

Selbstverständlich ist es zur Befestigung des rohrförmigen Bereichs 3 auch denkbar, sowohl am Schutzüberzug 1 als auch am Laparoskop 11 einen Magnetring oder in Kombination zu einen Magnetring einen Ring aus einem ferromagnetischen Material anzubringen. Zudem kann statt dieser magnetischen Befestigung, welche nur vorzugsweise als umlaufender Ring ausgebildet ist, auch eine andere Befestigungsart vorgesehen sein, wie beispielsweise ein Clip, eine Klammer oder Feder, insbesondere Federring, welcher an entsprechender Stelle in eine am Laparoskop 11 befindliche, vorzugsweise umlaufende Nut eingreift. Vorzugsweise befindet sich die Befestigungseinrichtung im proximalen Bereich des Laparoskops an Schaftanfang oder Bediengriff, insbesondere außerhalb der Eingriffstiefe des Schafts.

Wie aus Fig. 2 ersichtlich, weist der Laparoskopschaft 13, welcher in seinem überwiegenden Bereich starr ausgebildet ist, im distalen Bereich ein abwinkelbares bzw. flexibles Segment 15 auf, welches vorzugsweise innerhalb einer Ebene über Betätigung eines Steuerungsdrehgriffs 23 bis hin zu einem Winkel von +/- 100° abgewinkelt werden kann. An dieses flexible Element 15 schließt sich wiederum ein kurzer, beispielsweise etwa 20 mm langer, starrer Kopfbereich 17 an mit seinem stirnseitigen Kopfende 19 mit Deckglasoptik bzw. CCD-Sensor und LED-Lichtquelle. Die starren Bereiche, ausgenommen den Bereich 15, des Schaftes 13 sind hierbei meist aus Stahl gefertigt, wohingegen der flexible Bereich 15 im Bereich des Außenumfangs aus einem flexiblen Material wie Kunststoff oder Gummi besteht. Der innere Mechanismus für das Abwinkeln ist für die Erfindung nicht relevant und wird daher nicht näher dargestellt und erläutert.

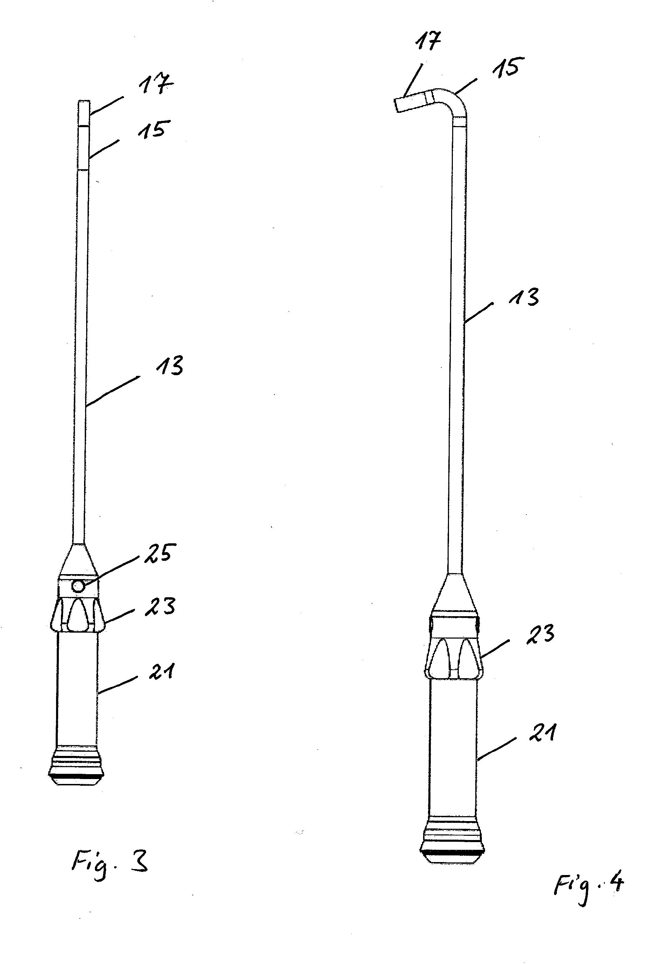

Das in Fig. 3 und Fig. 4 dargestellte Laparoskop zeigt dieses in seiner Null-Stellung (Fig. 3) ohne Abwinklung und in Fig. 4 im abgewinkelten Zustand von 100° (in der Zeichenebene nach links abgewinkelt), wobei durch Drehung des Drehgriffs 23 in entgegengesetzter Richtung die Abwinklung in entgegengesetzter Richtung stufenlos in der Zeichenebene ebenfalls nach 100° möglich ist.

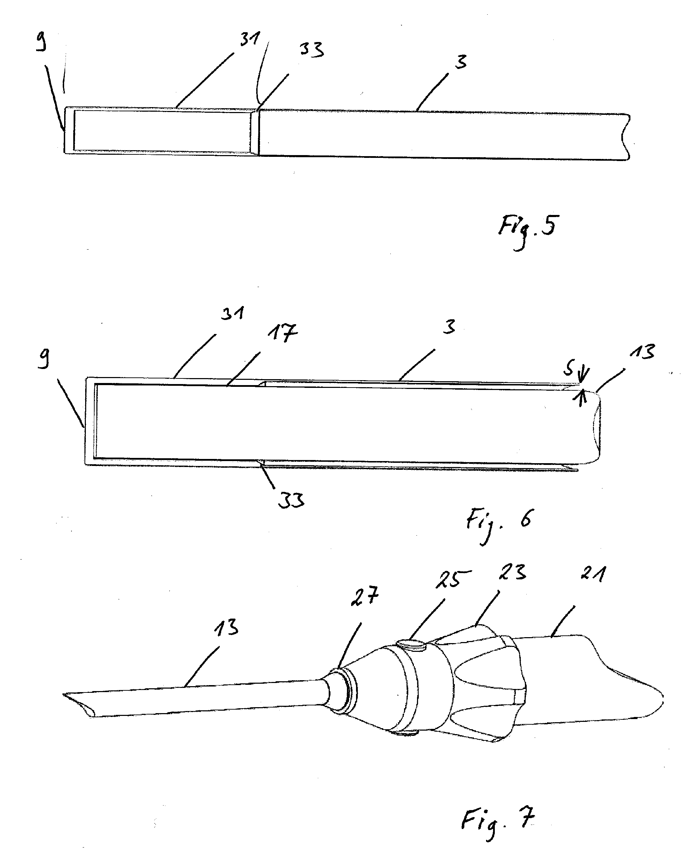

Der in Fig. 5 dargestellte Schnitt entlang der Länge des Schutzüberzugs 1 zeigt in abgebrochener Darstellung, dass der rohrförmige Bereich 3 bei gleichbleibendem Außendurchmesser gegenüber der Wandstärke im Kopfbereich 31 dünnwandig ausgebildet ist und nach dem Übergang bzw. der Stufe 33 der Kopfbereich 31 eine dickere Wandung aufweist.

Wie aus Fig. 6 ersichtlich, liegt daher im aufgeschobenen Zustand der Kopfbereich 31 spielfrei am Kopfbereich 17 des Laparoskops an, wohingegen der rohrförmige Bereich 3 in proximaler Richtung ab der Stufe 33 zum Schaft 13 des Laparoskops ein Spiel S von beispielsweise wenigen Zehntelmillimetern aufweist.

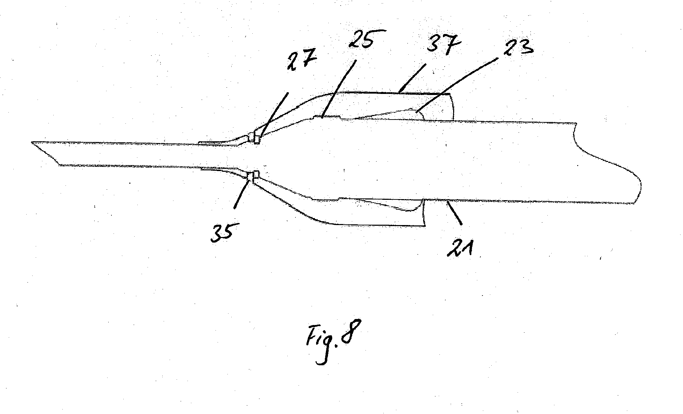

Wie in Fig. 7 und insbesondere Fig. 8 dargestellt, wird der rohrförmige Bereich 3 des Schutzüberzugs 1 am Schaft 13 durch eine Magnethalterung unter Spannung bzw. Zug fixiert. Hierzu kommen unter magnetischer Anziehungskraft die Magnetringe 35 und 27 oder ein Magnetring und ein Ring aus ferrromagnetischem Material aufeinander zu liegen, wobei der proximale Bereich des Schutzüberzugs 1 ab dieser Stelle, nämlich dem proximalen Bereich 37, vorzugsweise aus dünner, durchsichtiger Folie gefertigt, nicht unter Zug oder Spannung steht. Zudem weist dieser Bereich zum Handgriff 21 ein größeres Spiel, beispielsweise von einigen Zentimetern, auf, so dass durch diesen Bereich 37 hindurch von außen die Bedienelemente, wie Druckknopf 25 und Drehgriff 23, von außen bedienbar sind und dennoch das Laparoskop auch in diesem Bereich gegenüber äußeren Einflüssen dichtend geschützt ist.

Der rohrförmige bzw. schlauchförmige Bereich 3 kann in bevorzugter Ausgestaltung unter Zug dehnbar und wenigstens im Bereich des abwinkelbaren Segments 15 am distalen Ende (beispielsweise das letzte Drittel, Viertel oder Fünftel des Schafts) flexibel ausgebildet sein, so dass trotz einem distalen engen Anliegen ein Abwinkeln ermöglicht wird. Hierzu kann der Überzug 1 im Bereich des Schaftes 13 schlauchähnlich, insbesondere rohrähnlich und zumindest teilweise eng bzw. spielfrei anliegend ausgebildet sein.

In weiterer Ausgestaltung kann das Laparoskop statt der dargestellten stirnseitigen 0°-Optik (senkrecht zur Längsachse) eine Schräge von beispielsweise 30° aufweisen, um auf diese Weise selbst im ungebeugten Zustand (0° Beugung) eine seitliche Sicht zu ermöglichen. Bei dieser Ausgestaltung wäre es auch denkbar, statt einer Beugung in beide Richtungen (+/- 100°) nur eine Beugung in eine Richtung auszubilden, um beispielsweise bis zu 110° oder gar 150°, um sogar mit zusätzlicher 30°-Schräge eine bis zu 180°-Rücksicht zu ermöglichen.

Um bei dieser Ausführungsform mit 30°-Schräge (distal) ein richtiges Aufsetzen bzw. Aufschieben des Schutzüberzugs, insbesondere des rohrförmiges Bereichs 3, zu erleichtern, kann außen am Schutzüberzug 1, insbesondere im rohrförmigen Bereich 3, eine Markierung angebracht sein, welche mit einer Markierung am Laparoskop oder einem vorhandenen Element, beispielsweise dem Druckknopf 25, fluchtend in Eingriff gebracht werden muss. Selbstverständlich ist es auch denkbar als Befestigungseinrichtung Eingriffsmittel vorzusehen, welche ein ungewolltes Verdrehen des Schutzüberzugs (um dessen Längsachse) verhindern und/oder ein lagerichtiges Aufschieben gewährleisten. Beispielsweise kann hierfür am Laparoskop, insbesondere am Schaft oder Übergang von Schaft zum Bediengriff in Längsrichtung oder zumindest in einer anderen als hierzu senkrechten Richtung eine Nut vorgesehen sein, welche mit einer entsprechend am Innenumfang des Schutzüberzugs an entsprechender Stelle komplementär ausgebildeten Wulst beim Aufschieben (Längsführung) und/oder in der Endlage (Einrasten) in Eingriff kommt. Weiterhin ist es denkbar, statt einer Nut, auch eine Einkerbung, Ausnehmung, etc., welche nicht rotationssymmetrisch ausgebildet ist, in welche ein am Innenumfang an entsprechender Stelle ausgebildeter Vorsprung oder Nase des Schutzüberzugs in der gewünschten Endlage eingreift.

Hierdurch kann ein ungewolltes Verdrehen des rohrförmigen Bereichs vermieden werden und damit ein drehrichtiges Aufsetzen und Anliegen des Sichtfensters 9, welches ja ebenfalls eine 30°-Schräge aufweist, am Kopfende 19 gewährleistet werden. In bevorzugter Ausgestaltung ist zumindest der rohrförmige Bereich 3 aus Kunststoff gefertigt, wobei der rohrförmige Bereich 3 im aufgeschobenen Zustand im Bereich des abwinkelbaren Segments 15 flexibel, beispielsweise durch entsprechende Veränderung der Zusammensetzung des Kunststoffs oder durch Einbringung eines anderen entsprechend flexiblen Kunststoffs, ausgebildet ist, wohingegen der übrige Bereich des rohrförmigen Bereichs 3 im Wesentlichen formsteif ausgebildet ist, insbesondere in der Nähe der Stufe 33. Hierdurch kann beim Einführen in einen Trokar bzw. dessen Tubus eine Dichtwirkung zwischen Außenumfang, rohrförmigem Bereich an dieser Stelle und Innenumfang des Trokars bzw. dessen Tubus gesichert werden.

Selbstverständlich sind die vorstehend für beliebige Ausführungsformen erläuterten Befestigungseinrichtung zur lösbaren aber dennoch eindeutigen Fixierung der gewünschten Endlage und/oder zur rotationsrichtigen Längsführung für alle Ausführungsformen einzeln oder in Kombination oder in kinematischer Umkehr anwendbar.

## Patentansprüche

1. Schutzüberzug für ein Laparoskop (11),
**dadurch gekennzeichnet, dass**
a) der Schutzüberzug (1) in distaler Richtung gegen die Umgebung abgeschlossen und dichtend ausgebildet ist, um im aufgeschobenen Zustand im operativen Einsatz das Innere des Abdomens vor Verunreinigungen und/oder Infektionen und/oder das Laparoskop (11) vor Verunreinigungen zu schützen,
b) der Schutzüberzug (1) einen rohrförmigen Bereich (3) aufweist, welcher auf einen Schaft (13) des Laparoskops (11) geschoben werden kann,
c) der Schutzüberzug (1) wenigstens im Bereich des Schaftes (13) als geschlossene Hülle ausgebildet ist und
d) der Schutzüberzug (1) für die Optik des Laparoskops am distalen Kopfende (19) ein Sichtfenster (9) aufweist.

2. Schutzüberzug nach Anspruch 1, **dadurch gekennzeichnet, dass** der Schutzüberzug (1) im aufgeschobenen Zustand im Bereich eines abwinkelbaren Segments (15) des Schaftes (13) des Laparoskops (11) flexibel ausgebildet ist, um ein Abwinkeln des Schaftes (13) in diesem Bereich (15) zu ermöglichen.

3. Schutzüberzug nach Anspruch 1 oder 2, **dadurch gekennzeichnet, dass** der Schutzüberzug (1) im aufgeschobenen Zustand im Bereich des distalen Laparoskopkopfs (17) eng anliegend ausgebildet ist.

4. Schutzüberzug nach einem der vorhergehenden Ansprüche, **dadurch gekennzeichnet, dass** der Schutzüberzug (1) eine Befestigungseinrichtung (7) aufweist, mit welcher wenigstens der rohrförmige Bereich (3) unter Zug am Schaft (13) befestigbar ist, um eine ortsfeste Anordnung des Sichtfensters (9) relativ zur Optik und ein definiertes Anliegen des Sichtfensters (9) an der Optik zu ermöglichen.

5. Schutzüberzug nach Anspruch 4, **dadurch gekennzeichnet, dass** die Befestigungseinrichtung (7) als Magnetverschluss, ferromagnetischer Ring, Federring oder Clip ausgebildet ist, welcher mit einem entsprechend hierzu komplementär ausgebildeten Bereich (27) des Laparoskops zusammenwirkt, um eine lösbare definierte Befestigung zu ermöglichen.

6. Schutzüberzug nach einem der vorhergehenden Ansprüche, **dadurch gekennzeichnet, dass** der Schutzüberzug (1) im Bereich des Griffs (21) (dünn bzw.) flexibel und oder mit Spiel zu diesem Bereich (21) ausgebildet ist, um eine Bedienung der Bedienelemente (23, 25) des Laparoskops (11) durch den Schutzüberzug (1) hindurch zu ermöglichen.

7. Schutzüberzug nach einem der vorhergehenden Ansprüche, **dadurch gekennzeichnet, dass** das Sichtfenster (9) am distalen Ende des rohrförmigen Bereichs (3) als eingefügte Linse oder durchsichtiger Bereich ausgebildet ist.

8. Schutzüberzug nach einem der vorhergehenden Ansprüche, **dadurch gekennzeichnet, dass** der Schutzüberzug (1) aus Kunststoff und/oder Gummi gefertigt ist.

9. Laparoskop für einen aufschiebbaren und abnehmbaren Schutzüberzug (1) nach einem der vorhergehenden Ansprüche 4 bis 8,
a) wobei das Laparoskop (11) an seinem starren Schaft (13) unterhalb eines distalen Kopfes (17) ein abwinkelbares Segment (15) aufweist, so dass die Optik am Kopfende (19) ein einem Bereich von wenigstens 180° abwinkelbar ist,
**dadurch gekennzeichnet, dass**
b) das Laparoskop (11) eine Befestigungseinrichtung (27) aufweist, welche mit einer an oder in dem Schutzüberzug (1) angeordneten hierzu komplementär ausgebildeten Befestigungseinrichtung (7) zusammenwirkt, um den rohrförmigen Bereich (3) an dem Schaft (13) eindeutig und unter Spannung zu befestigen.

10. Anordnung aus einem Laparoskop und einem Schutzüberzug nach einem der Ansprüche 1 bis 8.
